# NEW EUROPEAN PATENT SPECIFICATION

(11) **EP 3 344 227 B2**
(45) Date of publication and mention of the opposition decision: **23.07.2025**
(45) Mention of the grant of the patent: 22.07.2020
(21) Application number: 16763478.1
(22) Date of filing: 01.09.2016
(51) Int. Cl.: A61K 8/39, A61Q 5/02, A61Q 5/12, A61K 8/81, A61K 8/86, A61K 8/44, A61K 8/46

(54) **COMPOSITION COMPRISING AT LEAST ONE ANIONIC SURFACTANT, AT LEAST TWO PARTICULAR NONIONIC SURFACTANTS, AT LEAST ONE AMPHOTERIC SURFACTANT AND AT LEAST ONE PARTICULAR CATIONIC POLYMER**
ZUSAMMENSETZUNG MIT MINDESTENS EINEM ANIONISCHEN TENSID, MINDESTENS ZWEI BESONDEREN NICHTIONISCHEN TENSIDEN, MINDESTENS EINEM AMPHOTEREN TENSID UND MINDESTENS EINEM KATIONISCHEN POLYMER
COMPOSITION COMPRENANT AU MOINS UN TENSIOACTIF ANIONIQUE, AU MOINS DEUX TENSIOACTIFS NON IONIQUES PARTICULIERS, AU MOINS UN TENSIOACTIF AMPHOTÈRE ET AU MOINS UN POLYMÈRE CATIONIQUE PARTICULIER

(30) Priority: 01.09.2015 FR 1558103
(43) Date of publication of application: 11.07.2018
(73) Proprietor: L'OREAL, 75008 Paris (FR)
(72) Inventor: MATHONNEAU, Estelle, 93400 Saint Ouen (FR); D'ARRAS, Marie-Florence, 93400 Saint Ouen (FR); LEBON-HIPOLITE, Emmanuelle, 94152 Chevilly-Larue (FR); SAMAIN, Henri, 91570 Bievres (FR); PLOS, Grégory, 93400 Saint Ouen (FR)
(74) Representative: Casalonga
(86) International application number: PCT/EP2016/070640
(87) International publication number: WO 2017/037187

(56) References cited:
- EP-A1- 1 132 079
- EP-A1- 1 557 155
- US-A1- 2006 020 145
- US-A1- 2010 249 004
- US-B1- 6 489 286
- DATABASE GNPD [online] MINTEL; June 2015 (2015-06-01), "Shampoo", XP002756643, Database accession no. 3207215

## Description

The present invention relates to a composition for cleansing and conditioning keratin fibres, in particular human keratin fibres such as the hair, comprising one or more anionic surfactants, two or more particular nonionic surfactants preferably in a total content of at least 3% by weight relative to the total weight of the composition, one or more amphoteric surfactants preferably in a total content of at least 2% by weight relative to the total weight of the composition and one or more cationic polymers having a cationic charge density of greater than or equal to 4 meq./g.

The invention also relates to a process for cleansing and conditioning keratin fibres and also to a use involving said composition.

It is common practice to use detergent compositions (such as shampoos) based essentially on standard surfactants especially of anionic, nonionic and/or amphoteric type, but more particularly of anionic type, for cleansing and/or washing keratin fibres such as the hair. These compositions are applied to wet hair and the foam generated by massaging or rubbing with the hands makes it possible, after rinsing with water, to remove the diverse types of soiling initially present on the hair or the skin. Such compositions are for example disclosed in the patent application EP 1 132 079.

These detergent compositions do, admittedly, have good washing power, but the cosmetic properties thereby imparted still remain to be improved, especially when they are applied to sensitized hair, i.e. hair that is generally damaged or embrittled by the action of external atmospheric agents, such as light and bad weather, and/or mechanical or chemical treatments, such as brushing, combing, dyeing, bleaching, permanent waving and/or relaxing.

Moreover, these compositions have a relatively aggressive nature since their application may result in the long run in more or less pronounced damage on the hair fibre associated in particular with the gradual removal of the lipids or proteins contained in or at the surface of said fibre.

Thus, it is often common practice to resort to care compositions using complementary cosmetic agents known as conditioning agents in order to improve the cosmetic properties of sensitized hair. These conditioning agents may, of course, also improve the cosmetic behaviour of natural hair.

These care compositions are especially hair conditioners, which may be in the form of hair gels or lotions or more or less thick creams.

However, the cationic surfactants used in such care compositions, especially in hair conditioners, are difficult to use in detergent compositions on account of their incompatibility with anionic surfactants.

In particular, their incompatibility with anionic surfactants may give rise to stabilization difficulties for the detergent compositions and have an impact on their working qualities, especially in terms of foaming power and/or start of foaming, as already mentioned in the patent application EP 1 557 155.

Furthermore, the presence of cationic surfactants in the detergent compositions may lead, after one or more applications to the hair, to heaviness or overcharging of the head of hair (which is known as the build-up effect), thus having a negative impact on the cosmetic properties imparted.

Moreover, such care compositions give the hair cosmetic properties that may fade out after the application of one or more non-treating shampoos.

There is thus a real need to develop compositions for cleansing and conditioning keratin fibres, which do not have all of the drawbacks mentioned above, i.e. which are capable of imparting improved cosmetic properties, after one or more applications, which are shampoo-resistant without making the head of hair charged or heavy, while at the same time maintaining good washing power and satisfactory working qualities.

This aim is achieved by the present invention, one subject of which is especially a cosmetic composition, preferably a hair composition, intended especially for cleansing and conditioning keratin fibres, in particular human keratin fibres such as the hair, comprising:
(a) one or more anionic surfactants,
(b) a mixture of a first nonionic surfactant chosen from saturated or unsaturated, linear or branched, oxyalkylenated C₈ to C₄₀ fatty alcohols and of a second nonionic surfactant, different from the first nonionic surfactant, chosen from saturated or unsaturated, linear or branched, oxyalkylenated C₈ to C₄₀ fatty alcohols, the mixture of nonionic surfactants being present in a total content of at least 3% by weight relative to the total weight of the composition,
(c) one or more amphoteric surfactants, preferably present in a total content of at least 2% by weight relative to the total weight of the composition, and
(d) one or more cationic polymers having a cationic charge density of greater than or equal to 4 meq./g.

Specifically, the composition according to the invention makes it possible to improve the cosmetic properties imparted to the keratin fibres, especially to the hair, preferably sensitized hair. In particular, the composition according to the invention makes it possible to improve the disentangling, the suppleness and also the feel of the hair, without a build-up effect.

It is thus possible to obtain adequate conditioning immediately on application, which may be shampoo-resistant, which does not make the hair feel charged, with a composition which develops an abundant, good-quality foam.

The invention may also make it possible to convey and deposit larger amounts of cationic polymers; finally, the compositions are of interest in the field of protecting the colour of dyed hair against washing (shampooing).

The composition according to the invention also has the advantage of being stable on storage both at room temperature (20-25°C) and at 45°C, especially as regards its visual aspect and/or its viscosity.

For the purposes of the present invention, the term "stable" refers to a composition which, after two months of storage at 25 to 45°C, shows no change in appearance, colour, odour or viscosity.

The composition may be transparent; it may also be pearlescent, where appropriate.

The composition according to the invention may also have satisfactory foaming power.

The composition according to the invention also makes it possible to apply conditioning agents, such as cationic polymers, in an amount sufficient to obtain adequate cosmetic conditioning properties.

Preferably, the composition according to the invention is non-colouring.

According to the present invention, the term "non-colouring composition" means a composition not containing any dye for keratin fibres, such as direct dyes or oxidation dye precursors (bases and/or couplers). If they are present, their content does not exceed 0.005% by weight, relative to the total weight of the composition. Specifically, at such a content, only the composition would be dyed, i.e. no dyeing effect would be observed on the keratin fibres.

The invention thus relates to a composition comprising the compounds (a), (b), (c) and (d) as defined above.

The present invention also relates to a process for washing and conditioning keratin fibres, in particular human keratin fibres such as the hair, comprising the application to said fibres of a composition according to the invention.

The invention also relates to the use of the composition according to the invention as a shampoo for washing and conditioning the hair.

Other subjects, characteristics, aspects and advantages of the invention will emerge even more clearly on reading the description and the examples that follow.

In the text hereinbelow, and unless otherwise indicated, the limits of a range of values are included in that range, in particular in the expressions "between" and "ranging from ... to ...".

Moreover, the expression "at least one" used in the present description is equivalent to the expression "one or more".

### Anionic surfactants

As indicated above, the composition comprises one or more anionic surfactants.

For the purposes of the present invention, the term "anionic surfactant" means a surfactant comprising, as ionic or ionizable groups, only anionic groups. These anionic groups are preferably chosen from the groups CO₂H, CO₂, SO₃H, SO₃⁻ , OSO₃H, OSO₃⁻, H₂PO₃, HPO₃⁻, PO₃²⁻, H₂PO₂, HPO₂⁻, PO₂²⁻, POH and PO⁻.

Preferably, the anionic surfactants used in the composition according to the invention are chosen from anionic surfactants comprising in their structure one or more sulfate and/or sulfonate and/or phosphate and/or carboxylate groups, and/or mixtures thereof.

Preferentially, the composition according to the invention comprises one or more carboxylic alkyl ether anionic surfactants.

More preferentially, the composition according to the invention comprises a mixture of anionic surfactants and especially one or more anionic surfactants comprising in their structure one or more sulfate and/or sulfonate and/or phosphate groups and one or more carboxylic alkyl ether anionic surfactants.

The anionic surfactant(s) comprising in their structure one or more sulfate and/or sulfonate and/or phosphate groups may be oxyethylenated and/or oxypropylenated. The total average number of ethylene oxide (EO) and/or propylene oxide (PO) groups may then range from 1 to 50 and especially from 2 to 10.

The anionic surfactant(s) comprising in their structure one or more sulfate and/or sulfonate and/or phosphate groups may be chosen from alkyl sulfates, alkylamido sulfates, alkyl ether sulfates, alkylamido ether sulfates, alkylaryl ether sulfates, alkyl ether sulfosuccinates, acyl isethionates and methyl acyl taurates, olefin sulfonates, and salts thereof; the alkyl or acyl group of all these various compounds preferably containing from 8 to 24 carbon atoms, and the aryl group preferably denoting a phenyl or benzyl group.

Among the anionic surfactants comprising in their structure one or more sulfate and/or sulfonate and/or phosphate groups, it is preferred to use one or more anionic sulfate surfactants, preferentially chosen from C₈-C₁₄ and more particularly C₁₂-C₁₄ alkyl ether sulfates, and/or from the olefin sulfonates.

Preferably, the anionic surfactant(s) comprising in their structure one or more sulfate and/or sulfonate and/or phosphate groups are in the form of salts, and in particular of alkaline salts, especially sodium salts, ammonium salts, amine salts, including amino alcohol salts, and/or magnesium salts. These salts preferably comprise from 2 to 5 ethylene oxide groups.

Among these salts, sodium, triethanolamine, magnesium or ammonium (C₁₂-C₁₄)alkyl sulfates and/or sodium, ammonium or magnesium (C₁₂-C₁₄)alkyl ether sulfates, which are oxyethylenated, for example with 1 or 2.2 mol of ethylene oxide or sodium olefin sulfonates, are more preferably used.

Better still, the surfactant(s) comprising in their structure one or more sulfate and/or sulfonate and/or phosphate groups are chosen from sodium, ammonium or magnesium (C₁₂-C₁₄)alkyl ether sulfates oxyethylenated with 2.2 mol of ethylene oxide, as sold under the name Texapon N702 by the company Cognis.

Among the anionic surfactant(s) comprising in their structure one or more sulfate and/or sulfonate and/or phosphate groups, it is preferred to use sodium or ammonium lauryl ether sulfates or sodium olefin sulfonates.

The carboxylic alkyl ether anionic surfactant(s) that may be used according to the invention preferably comprise a C₆-C₂₄ alkyl chain.

The carboxylic alkyl ether anionic surfactant(s) may be chosen, alone or as mixtures, from:
- (C₆-C₂₄)alkyl ether carboxylic acids,
- (C₆-C₂₄)alkylaryl ether carboxylic acids,
- (C₆-C₂₄)alkylamido ether carboxylic acids,
- and salts thereof.

The carboxylic alkyl ether anionic surfactant(s) may be oxyalkylenated, preferably oxyethylenated and/or oxypropylenated. The total average number of alkylene oxide groups then preferably ranges from 2 to 50, in particular from 2 to 24 and better still from 2 to 15.

When the carboxylic alkyl ether anionic surfactant(s) are oxyalkylenated, they preferably comprise from 2 to 50 alkylene oxide groups and in particular from 2 to 50 ethylene oxide (EO) groups.

Preferably, the carboxylic alkyl ether anionic surfactant(s) are neutralized with one or more salts. The salts are chosen in particular from alkaline salts and especially sodium salts, ammonium salts, amine salts, including amino alcohol salts such as triethanolamine or monoethanolamine salts, and magnesium salts.

The polyethoxylated carboxylic anionic surfactants more preferably used are those corresponding to formula (I) below:

R₁(OC₂H₄)ₙOCH₂COOA (I)

in which:
R₁ represents a linear or branched C₈-C₂₂ alkyl or alkenyl group or mixture of groups, a (C₈-C₉)alkylphenyl group, a group R₂CONH-CH₂-CH₂- with R₂ denoting a linear or branched C₁₁-C₂₁ alkyl or alkenyl group,
n is an integer or decimal number (average value) that may range from 2 to 24 and preferably from 2 to 15,
A denotes H, NH₄, Na, K, Li, Mg or a monoethanolamine or triethanolamine residue. Mixtures of compounds of formula (I) may also be used, in particular mixtures in which the groups R₁ are different.

Preferably, R₁ denotes a group or a mixture of groups chosen from C₁₂-C₁₄ alkyl, cocoyl, oleyl, nonylphenyl and octylphenyl groups; A denotes a hydrogen or sodium atom; and n ranges from 2 to 20 and preferably from 2 to 10.

Even more preferentially, use is made of compounds of formula (I) in which R₁ denotes a C₁₂ alkyl group, A denotes a hydrogen or sodium atom and n ranges from 2 to 10.

Among the commercial products that may preferably be used are the products sold by the company KAO under the names:
Akypo^{®} NP 70 (R₁ = nonylphenyl, n = 7, A = H)
Akypo^{®} NP 40 (R₁ = nonylphenyl, n = 4, A = H)
Akypo^{®} OP 40 (R₁ = octylphenyl, n = 4, A = H)
Akypo^{®} OP 80 (R₁ = octylphenyl, n = 8, A = H)
Akypo^{®} OP 190 (R₁ = octylphenyl, n = 19, A = H)
Akypo^{®} RLM 38 (R₁ = (C₁₂-C₁₄)alkyl, n = 4, A = H)
Akypo^{®} RLM 38 NV (R₁ = (C₁₂-C₁₄)alkyl, n = 4, A = Na)
Akypo^{®} RLM 45 CA (R₁ = (C₁₂-C₁₄)alkyl, n = 4.5, A = H)
Akypo^{®} RLM 45 NV (R₁ = (C₁₂-C₁₄)alkyl, n = 4.5, A = Na)
Akypo^{®} RLM 100 (R₁ = (C₁₂-C₁₄)alkyl, n = 10, A = H)
Akypo^{®} RLM 100 NV (R₁ = (C₁₂-C₁₄)alkyl, n = 10, A = Na)
Akypo^{®} RLM 130 (R₁ = (C₁₂-C₁₄)alkyl, n = 13, A = H)
Akypo^{®} RLM 160 NV (R₁ = (C₁₂-C₁₄)alkyl, n = 16, A = Na)
or by the company Sandoz under the names:
   Sandopan DTC-Acid (R₁ = (C₁₃)alkyl, n = 6, A = H)
   Sandopan DTC (R₁ = (C₁₃)alkyl, n = 6, A = Na)
   Sandopan LS 24 (R₁ = (C₁₂-C₁₄)alkyl, n = 12, A = Na)
   Sandopan JA 36 (R₁ = (C₁₃)alkyl, n = 18, A = H),
   and more particularly the products sold under the following names:
      Akypo^{®} RLM 45 (INCI: Laureth-5 carboxylic acid)
      Akypo^{®} RLM 100
      Akypo^{®} RLM 38.

Among the carboxylic alkyl ether anionic surfactants, use is preferably made of lauryl ether carboxylic acids or sodium lauryl ether carboxylates.

Preferably, the anionic surfactants are chosen from sulfate anionic surfactants, chosen especially from C₈-C₁₄ alkyl ether sulfates and carboxylic alkyl ether surfactants.

Preferentially, the composition according to the invention comprises one or more carboxylic alkyl ether surfactants corresponding to formula (I).

More preferentially, the composition according to the invention comprises a mixture of sulfate anionic surfactants, chosen especially from C₈-C₁₄ and more particularly C₁₂-C₁₄ alkyl ether sulfates, and carboxylic alkyl ether surfactants corresponding to formula (I) as described previously.

The anionic surfactant(s) may be present in the composition according to the invention in a total content ranging from 1% to 20% by weight, preferably in a content ranging from 2% to 18% by weight and better still from 4% to 15% by weight relative to the total weight of the composition.

### Nonionic surfactants

As indicated above, the composition comprises a mixture of nonionic surfactants the composition comprises at least a first nonionic surfactant chosen from saturated or unsaturated, linear or branched, oxyalkylenated C₈ to C₄₀ fatty alcohols and at least a second nonionic surfactant, different from the first nonionic surfactant, chosen from (i) saturated or unsaturated, linear or branched, oxyalkylenated C₈ to C₄₀ fatty alcohols.

The nonionic surfactant(s) present in the composition according to the invention are described, for example, in the Handbook of Surfactants by M.R. Porter, published by Blackie & Son (Glasgow and London), 1991, pp. 116-178.

The oxyalkylene units of the nonionic surfactants are more particularly oxyethylene or oxypropylene units, or their combination, preferably oxyethylene units.

The number of moles of ethylene oxide and/or of propylene oxide preferably ranges from 1 to 250, more particularly from 2 to 100 and better still from 2 to 50.

Advantageously, the two nonionic surfactants according to the invention do not comprise any oxypropylene units.

Preferably, the saturated or unsaturated, linear or branched, oxyethylenated C₈-C₄₀ fatty alcohols comprise from 1 to 100 mol of ethylene oxide, in particular from 2 to 50 and more particularly from 2 to 40 mol of ethylene oxide and preferably comprise one or two fatty chains.

Thus, the oxyethylenated fatty alcohols according to the invention are advantageously chosen from those of formula R-(O-CH₂CH₂)ₙ-OH, in which R is a saturated or unsaturated, linear or branched C₈ to C₄₀ hydrocarbon-based radical, preferably a linear or branched C₈ to C₄₀ alkyl radical; especially of C₈-C₂₄, or even C₈-C₂₀, and better still C₁₀-C₁₈, and
n is an integer ranging from 1 to 100, in particular from 2 to 50, or even from 2 to 40, and better still from 3 to 20.

More preferentially, the saturated or unsaturated, linear or branched, oxyethylenated C₈ to C₄₀ fatty alcohols comprise from 3 to 20 mol of ethylene oxide and comprising preferably at least one C₈-C₂₀ and especially C₁₀-C₁₈ alkyl chain, and correspond especially to lauryl alcohol containing 4 mol of ethylene oxide (INCI name: Laureth-4) and lauryl alcohol containing 12 mol of ethylene oxide (INCI name: Laureth-12).

According to a first embodiment, the composition according to the invention comprises at least two saturated or unsaturated, linear or branched, oxyalkylenated C₈ to C₄₀ fatty alcohols, which are different from each other.

Preferably, said two oxyethylenated fatty alcohols are chosen from those of formula R-(O-CH₂CH₂)ₙ-OH, in which R is a saturated or unsaturated, linear or branched C₈ to C₄₀ hydrocarbon-based radical, preferably a linear or branched C₈ to C₄₀ alkyl radical; especially of C₈-C₂₄, or even C₈-C₂₀, and better still C₁₀-C₁₈, and n is an integer ranging from 1 to 100, in particular from 2 to 50, or even from 2 to 40, and better still from 3 to 20.

The composition according to the invention comprises the nonionic surfactants in a total content of at least 3% by weight, preferably in a content ranging from 3% to 20% by weight, more preferentially in a content ranging from 4% to 15% by weight relative to the total weight of the composition, better still from 4.5% to 12% by weight.

### Amphoteric surfactants

As indicated above, the composition comprises one or more amphoteric surfactants which are preferably present in a total content of greater than or equal to at least 2% by weight relative to the total weight of the composition, especially greater than or equal to 3% by weight.

In particular, the amphoteric or zwitterionic surfactant(s), which are preferably non-silicone, used in the composition according to the present invention may especially be derivatives of optionally quaternized aliphatic secondary or tertiary amines, in which derivatives the aliphatic group is a linear or branched chain comprising from 8 to 22 carbon atoms, said amine derivatives containing at least one anionic group, for instance a carboxylate, sulfonate, sulfate, phosphate or phosphonate group.

Mention may in particular be made of (C₈-C₂₀)alkyl betaines, (C₈-C₂₀)alkyl sulfobetaines, (C₈-C₂₀)alkylamido(C₃-C₈)alkyl betaines and (C₈-C₂₀)alkylamido(C₆-C₈)alkyl sulfobetaines.

Among the optionally quaternized derivatives of secondary or tertiary aliphatic amines that can be used, as defined above, mention may also be made of the compounds with respective structures (II) and (III) below:

Rₐ-CONHCH₂CH₂-N⁺(R_{b})(R_{c})-CH₂COO⁻, M⁺, X⁻ (II)

in which formula:
- Rₐ represents a C₁₀ to C₃₀ alkyl or alkenyl group derived from an acid RₐCOOH preferably present in hydrolysed coconut oil, or a heptyl, nonyl or undecyl group;
- R_{b} represents a beta-hydroxyethyl group; and
- R_{c} represents a carboxymethyl group;
- M⁺ represents a cationic counterion resulting from an alkali metal or alkaline-earth metal, such as sodium, an ammonium ion or an ion resulting from an organic amine; and
- X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;

R_{a'}-CONHCH₂CH₂-N(B)(B') (III)

in which formula:
- B represents the group -CH₂CH₂OX;
- B' represents the group -(CH₂)_{z}Y', with z = 1 or 2;
- X' represents the group -CH₂COOH, -CH₂-COOZ', -CH₂CH₂COOH, -CH₂CH₂-COOZ', or a hydrogen atom;
- Y' represents the group -COOH, -COOZ' or -CH₂CH(OH)SO₃H or the group CH₂CH(OH)SO₃-Z';
- Z' represents a cationic counterion resulting from an alkali metal or alkaline-earth metal, such as sodium, an ammonium ion or an ion resulting from an organic amine;
- R_{a'} represents a C₁₀ to C₃₀ alkyl or alkenyl group of an acid R_{a'}-COOH which is preferably present in coconut oil or in hydrolysed linseed oil, or an alkyl group, especially a C₁₇ group, and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Use may also be made of the compounds of formula (IV):

R_{a"}-NHCH(Y")-(CH₂)ₙCONH(CH₂)_{n'}-N(R_{d})(Rₑ) (IV)

in which formula:
- Y" represents the group -COOH, -COOZ" or -CH₂-CH(OH)SO₃H or the group CH₂CH(OH)SO₃-Z";
- R_{d} and Rₑ represent, independently of each other, a C₁ to C₄ alkyl or hydroxyalkyl radical;
- Z" represents a cationic counterion resulting from an alkali metal or alkaline-earth metal, such as sodium, an ammonium ion or an ion resulting from an organic amine;
- R_{a"} represents a C₁₀-C₃₀ alkyl or alkenyl group of an acid R_{a"}-COOH which is preferably present in coconut oil or in hydrolysed linseed oil;
- n and n' denote, independently of each other, an integer ranging from 1 to 3.

Mention may be made, among the compounds of formula (II), of the compound classified in the CTFA dictionary under the name sodium diethylaminopropyl cocoaspartamide and sold by Chimex under the name Chimexane HB.

These compounds can be used alone or as mixtures.

Among the amphoteric or zwitterionic surfactants mentioned above, use is preferably made of (C₈-C₂₀)alkylbetaines such as cocoylbetaine, (C₈-C₂₀)alkylamido(C₃-C₈)alkylbetaines such as cocamidopropylbetaine, and mixtures thereof, and the compounds of formula (IV) such as the sodium salt of diethylaminopropyl laurylaminosuccinamate (INCI name: sodium diethylaminopropyl cocoaspartamide).

Preferentially, the amphoteric or zwitterionic surfactants are chosen from (C₈-C₂₀)alkylamido(C₃-C₈)alkylbetaines such as cocamidopropyl betaine.

Preferably, the amphoteric surfactant(s) are present in a total content ranging from 2% to 20% by weight, more preferentially in a content ranging from 3% to 15% by weight and better still from 3.5% to 10% by weight relative to the total weight of the composition.

### Cationic polymers

The cosmetic composition also comprises one or more cationic polymers with a cationic charge density of greater than or equal to 4 milliequivalents/gram (meq./g).

The cationic charge density of a polymer corresponds to the number of moles of cationic charges per unit mass of polymer under conditions in which it is totally ionized. It may be determined by calculation if the structure of the polymer is known, i.e. the structure of the monomers constituting the polymer and their molar proportion or weight proportion. It may also be determined experimentally by the Kjeldahl method.

For the purposes of the present invention, the term "cationic polymer" denotes any polymer containing cationic groups and/or groups that can be ionized into cationic groups.

The cationic polymers with a charge density of greater than or equal to 4 meq./g that are preferred are chosen from those that contain units comprising primary, secondary, tertiary and/or quaternary amine groups that can either form part of the main polymer chain or be borne by a side substituent directly attached thereto.

The cationic polymers used generally have a number-average molecular mass of between 500 and 5 × 10⁶ approximately and preferably between 10³ and 3 × 10⁶ approximately.

Among the cationic polymers having a charge density of greater than or equal to 4 meq./g, mention may more particularly be made of polymers of the polyamine, polyaminoamide and polyquaternary ammonium type.

These are known products. They are especially described in French patents 2 505 348 and 2 542 997. Among said polymers, mention may be made of:
(1) Homopolymers or copolymers derived from acrylic or methacrylic esters or amides and comprising at least one of the units of formula (V), (VI), (VII) or (VIII) below: 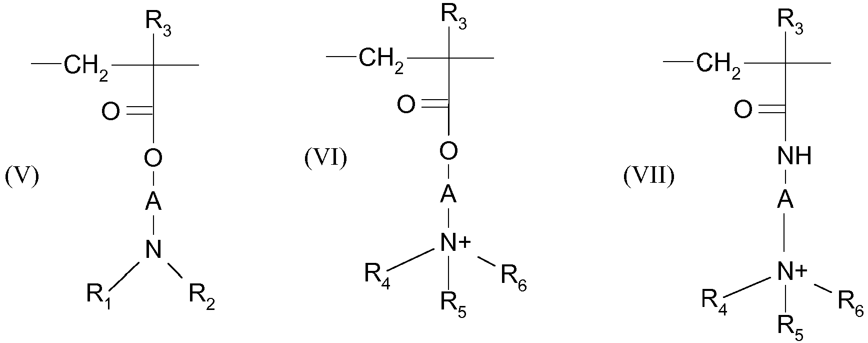 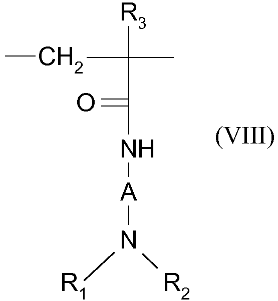 in which:
   R₃, which may be identical or different, denote a hydrogen atom or a CH₃ radical;
   A, which may be identical or different, represent a linear or branched alkyl group of 1 to 6 carbon atoms, preferably 2 or 3 carbon atoms, or a hydroxyalkyl group of 1 to 4 carbon atoms;
   R₄, R₅ and R₆, which may be identical or different, represent an alkyl group containing from 1 to 18 carbon atoms or a benzyl radical and preferably an alkyl group containing from 1 to 6 carbon atoms;
   R₁ and R₂, which may be identical or different, represent hydrogen or an alkyl group containing from 1 to 6 carbon atoms, and preferably methyl or ethyl;
   X denotes an anion derived from a mineral or organic acid, such as a methosulfate anion or a halide such as chloride or bromide.

Mention may be made in particular of the ethyltrimethylammonium methacrylate chloride homopolymer.

The polymers of family (1) can also contain one or more units derived from comonomers which may be chosen from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters.

Thus, among these polymers of family (1), mention may be made of:
- copolymers of acrylamide and of dimethylaminoethyl methacrylate quaternized with dimethyl sulfate or with a dimethyl halide,
- copolymers of acrylamide and of methacryloyloxyethyltrimethylammonium chloride,
- the copolymer of acrylamide and of methacryloyloxyethyltrimethylammonium methosulfate,
- quaternized or non-quatemized vinylpyrrolidone/dialkylaminoalkyl acrylate or methacrylate copolymers,

These polymers are described in detail in French patents 2 077 143 and 2 393 573,
- dimethylaminoethyl methacrylate/vinylcaprolactam/vinylpyrrolidone terpolymers,
- vinylpyrrolidone/methacrylamidopropyldimethylamine copolymers,
- quaternized vinylpyrrolidone/dimethylaminopropylmethacrylamide copolymers,
- the crosslinked polymers of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, in particular methylenebisacrylamide. Use may more particularly be made of a crosslinked acrylamide/methacryloyloxyethyltrimethylammonium chloride copolymer (20/80 by weight) in the form of a dispersion containing 50% by weight of said copolymer in mineral oil. This dispersion is sold under the name Salcare^{®} SC 92 by the company Ciba. Use may also be made of a crosslinked homopolymer of methacryloyloxyethyltrimethylammonium chloride comprising approximately 50% by weight of the homopolymer in mineral oil or in a liquid ester. These dispersions are sold under the names Salcare^{®} SC 95 and Salcare^{®} SC 96 by the company Ciba.

(2) Cyclopolymers of alkyldiallylamine or of dialkyldiallylammonium, such as homopolymers or copolymers comprising, as main constituent of the chain, units corresponding to formula (IX) or (X): 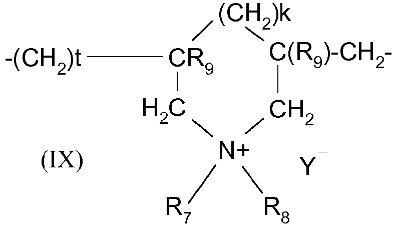 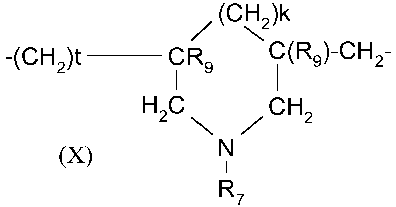 in which formulae k and t are equal to 0 or 1, the sum k + t being equal to 1; R₉ denotes a hydrogen atom or a methyl radical; R₇ and R₈, independently of each other, denote an alkyl group containing from 1 to 6 carbon atoms, a hydroxyalkyl group in which the alkyl group preferably contains 1 to 5 carbon atoms, or a lower (C₁-C₄) amidoalkyl group, or R₇ and R₈ may denote, together with the nitrogen atom to which they are attached, heterocyclic groups, such as piperidyl or morpholinyl; R₇ and R₈, independently of each other, preferably denote an alkyl group containing from 1 to 4 carbon atoms; Y⁻ is an anion such as bromide, chloride, acetate, borate, citrate, tartrate, bisulfate, bisulfite, sulfate or phosphate. These polymers are in particular described in French patent 2 080 759 and in its Certificate of Addition 2 190 406.

Among the polymers defined above, mention may be made more particularly of the dimethyldiallylammonium chloride homopolymer sold under the name Merquat 100 by the company Nalco (and its homologues of low weight-average molecular mass).

(3) The quaternary diammonium polymer containing repeating units corresponding to formula (XI): 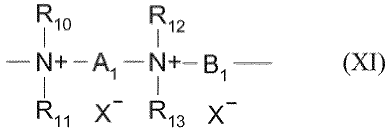 in which formula (XI)
R₁₀, R₁₁, R₁₂ and R₁₃, which may be identical or different, represent aliphatic, alicyclic or arylaliphatic radicals containing from 1 to 6 carbon atoms or lower hydroxyalkylaliphatic radicals, or alternatively R₁₀, R₁₁, R₁₂ and R₁₃, together or separately, constitute, with the nitrogen atoms to which they are attached, heterocycles optionally containing a second heteroatom other than nitrogen, or alternatively R₁₀, R₁₁, R₁₂ and R₁₃ represent a linear or branched C₁-C₆ alkyl radical substituted with a nitrile, ester, acyl or amide group or a group -CO-O-R₁₄-D or -CO-NH-R₁₄-D where R₁₄ is an alkylene and D is a quaternary ammonium group;
A1 and B1 represent polymethylene groups containing from 2 to 8 carbon atoms, which may be linear or branched and saturated or unsaturated and may contain, bonded to or intercalated in the main chain, one or more aromatic rings, or one or more oxygen or sulfur atoms, or sulfoxide, sulfone, disulfide, amino, alkylamino, hydroxyl, quaternary ammonium, ureido, amide or ester groups, and
X⁻ denotes an anion derived from a mineral or organic acid;
A1, R₁₀ and R₁₂ can form, with the two nitrogen atoms to which they are attached, a piperazine ring; in addition, if A1 denotes a linear or branched, saturated or unsaturated alkylene or hydroxyalkylene radical, B1 can also denote a group - (CH₂)n-CO-D-OC-(CH₂)n- in which D denotes:
   a) a glycol residue of formula: -O-Z-O-, where Z denotes a linear or branched hydrocarbon-based radical or a group corresponding to one of the following formulae:

      -(CH₂-CH₂-O)x-CH₂-CH₂-

      -[CH₂-CH(CH₃)-O]y-CH₂-CH(CH₃)-

      where x and y denote an integer from 1 to 4, representing a defined and unique degree of polymerization or any number from 1 to 4 representing an average degree of polymerization;
   b) a bis-secondary diamine residue, such as a piperazine derivative;
   c) a bis-primary diamine residue of formula: -NH-Y-NH-, where Y denotes a linear or branched hydrocarbon-based radical, or else the divalent radical

      -CH₂-CH₂-S-S-CH₂-CH₂- ;
   d) a ureylene group of formula: -NH-CO-NH-.

Preferably, X⁻ is an anion, such as chloride or bromide.

These polymers have a number-average molecular mass generally between 1000 and 100 000.

Polymers of this type are especially described in French patents 2 320 330, 2 270 846, 2 316 271, 2 336 434 and 2 413 907 and US patents 2 273 780, 2 375 853, 2 388 614, 2 454 547, 3 206 462, 2 261 002, 2 271 378, 3 874 870,4 001 432, 3 929 990, 3 966 904, 4 005 193, 4 025 617, 4 025 627, 4 025 653, 4 026 945 and 4 027 020.

It is more particularly possible to use polymers that are formed from repeating units corresponding to formula (XII) below: 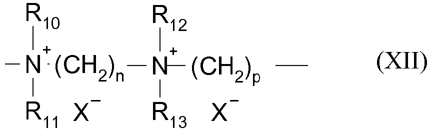 in which R₁₀, R₁₁, R₁₂ and R₁₃, which may be identical or different, denote an alkyl or hydroxyalkyl radical containing from 1 to 4 carbon atoms approximately, n and p are integers ranging from 2 to 8 approximately, and X₋ is an anion derived from a mineral or organic acid. Mention may be made in particular of Mexomer PO sold by the company Chimex.

(4) Polyquaternary ammonium polymers formed from repeating units of formula (XIII): 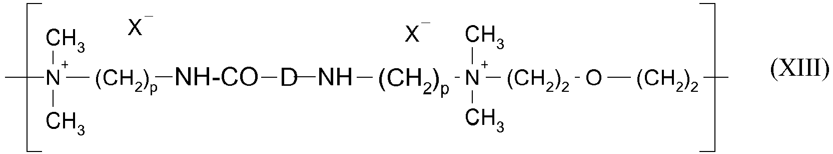 in which p denotes an integer ranging from 1 to 6 approximately, D may be nothing or may represent a group -(CH₂)ᵣ-CO- in which r denotes a number equal to 4 or 7, and X⁻ is an anion.

Such polymers may be prepared according to the processes described in US patents 4 157 388, 4 702 906 and 4 719 282. They are especially described in patent application EP-A-122 324.

Among these polymers, examples that may be mentioned include the products Mirapol A 15, Mirapol AD1, Mirapol AZ1 and Mirapol 175 sold by the company Miranol.

(5) Quaternary polymers of vinylpyrrolidone and of vinylimidazole.

Other cationic polymers that may be used in the context of the invention are polyalkyleneimines, in particular polyethyleneimines, polymers containing vinylpyridine or vinylpyridinium units, condensates of polyamines and of epichlorohydrin, polyquaternary ureylenes and chitin derivatives.

Hence the cationic polymers according to the invention are chosen preferentially from polymers, with a charge density of greater than or equal to 4 meq./g, belonging to families (1) to (5) as described above.

Among all the cationic polymers that may be used in the context of the present invention, it is preferred to use polymers of the family (2).

Preferably, the cationic polymer(s) are chosen from cyclopolymers of alkyldiallylamine or of dialkyldiallylammonium, such as homopolymers or copolymers comprising, as main constituent of the chain, units corresponding to formula (IX) or (X).

Preferentially, the cationic polymers are chosen from dialkyldiallylammonium halide homopolymers, more particularly the dialkyldiallylammonium chloride homopolymer (INCI name: Polyquaternium-6) sold under the name Merquat^{®} 100 by the company Lubrizol.

The content of cationic polymer(s) with a charge density of greater than or equal to 4 meq./g in the composition according to the invention may range from 0.05% to 5% by weight relative to the total weight of the composition, preferably from 0.1% to 3% by weight and more preferentially from 0.2% to 1.5% by weight relative to the total weight of the composition.

The total content of cationic polymer(s) in the composition according to the invention may range from 0.05% to 5% by weight relative to the total weight of the composition, preferably from 0.1% to 3% by weight and more preferentially from 0.2% to 1.5% by weight relative to the total weight of the composition.

According to one embodiment, the composition according to the invention comprises:
- one or more carboxylic alkyl ether anionic surfactants, preferably of formula (I) as defined above,
- a mixture of nonionic surfactants, preferably in a total content of at least 3% by weight relative to the total weight of the composition, comprising:
   (i) a first nonionic surfactant chosen from among one or more oxyethylenated C₈ to C₄₀ fatty alcohols comprising from 2 to 50 mol of ethylene oxide and preferably comprising at least one C₈-C₂₀ alkyl chain, and
   (ii) a second nonionic surfactant chosen from one or more oxyethylenated C₈ to C₄₀ fatty alcohols comprising from 2 to 50 mol of ethylene oxide and preferably comprising at least one C₈-C₂₀ alkyl chain,
- one or more amphoteric surfactants, preferably in a total content of at least 2% by weight relative to the total weight of the composition, comprising one or more surfactants chosen from (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines, and
- one or more cationic polymers with a cationic charge density of greater than or equal to 4 meq./g, chosen from dialkyldiallylammonium halide homopolymers.

According to a second embodiment, the composition according to the invention comprises:
- one or more anionic surfactants chosen from sulfate anionic surfactants, especially from C₈-C₁₄ alkyl ether sulfates and carboxylic alkyl ether surfactants,
- a mixture of nonionic surfactants, preferably in a total content of at least 3% by weight relative to the total weight of the composition, comprising:
   (i) a first nonionic surfactant chosen from one or more oxyethylenated C₈ to C₄₀ fatty alcohols comprising from 2 to 50 mol of ethylene oxide and comprising preferably at least one C₈-C₂₀ alkyl chain, and
   (ii) a second nonionic surfactant chosen from one or more oxyethylenated C₈ to C₄₀ fatty alcohols comprising from 2 to 50 mol of ethylene oxide and preferably comprising at least one C₈-C₂₀ alkyl chain,
- one or more amphoteric surfactants, preferably in a total content of at least 2% by weight relative to the total weight of the composition, chosen from (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines, and
- one or more cationic polymers with a cationic charge density of greater than or equal to 4 meq./g, chosen from diallyldialkylammonium halide homopolymers.

In accordance with this second embodiment, the composition preferentially comprises a mixture of sulfate anionic surfactants, chosen especially from C₈-C₁₄ and more particularly C₁₂-C₁₄ alkyl ether sulfates, and carboxylic alkyl ether surfactants corresponding to formula (I) as defined previously.

In accordance with these two embodiments, the amphoteric surfactants are preferably chosen from (C₈-C₂₀)alkylamido(C₃-C₈)alkylbetaines.

In accordance with these two embodiments, the mixture of nonionic surfactants comprises two or more different oxyethylenated C₈ to C₄₀ fatty alcohols comprising from 2 to 50 mol of ethylene oxide and comprising preferably at least one C₈-C₂₀ alkyl chain.

The composition according to the invention may comprise water or a mixture of water and one or more cosmetically acceptable solvents selected from C₁-C₄ lower alcohols, such as ethanol, isopropanol, tert-butanol or n-butanol; polyols such as glycerol, propylene glycol and polyethylene glycols; and mixtures thereof.

The pH of the compositions according to the invention generally ranges from 3 to 10, preferably from 3.5 to 7 and better still from 4 to 5.5.

The composition according to the invention may also comprise one or more standard additives that are well known in the art, such as natural or synthetic thickeners or viscosity regulators; C₁₂-C₃₀ fatty alcohols; ceramides; fatty esters such as isopropyl myristate, myristyl myristate, cetyl palmitate and stearyl stearate; mineral, plant or synthetic oils such as α-olefins or avocado oil, rapeseed oil, apricot oil, camelina oil or liquid petroleum jelly; vitamins or provitamins; amphoteric or nonionic or anionic polymers; pH stabilizers, preserving agents; dyes; fragrances.

The thickener(s) may be chosen from cellulose-based thickeners, for example hydroxyethylcellulose, hydroxypropylcellulose and carboxymethylcellulose, guar gum and derivatives thereof, for example the hydroxypropyl guar sold by the company Rhodia under the reference Jaguar HP 105, gums of microbial origin, such as xanthan gum and scleroglucan gum, synthetic thickeners such as crosslinked acrylic acid or acrylamidopropanesulfonic acid homopolymers, for example Carbomer, nonionic, anionic, cationic or amphoteric associative polymers, such as the polymers sold under the names Pemulen TR1 or TR2 by the company Goodrich, Salcare SC90 by the company Ciba, Aculyn 22, 28, 33, 44 or 46 by the company Röhm & Haas, and Elfacos T210 and T212 by the company Akzo.

Mention may be made, among the amphoteric polymers used, of acrylamide/methacrylamidopropyltrimethylammonium chloride/acrylic acid terpolymers. Such polymers are listed in the CTFA International Cosmetic Ingredient Dictionary, 10th edition 2004, as Polyquaternium 53. Corresponding products are especially sold under the name Merquat 2003 by the company Lubrizol.

A person skilled in the art will take care to select the optional additives and the amount thereof such that they do not harm the properties of the compositions of the present invention.

These additives are generally present in the composition according to the invention in an amount ranging from 0 to 20% by weight relative to the total weight of the composition.

The compositions in accordance with the invention may be used as shampoos for washing and conditioning the hair, and they are preferably applied in this case to wet hair in amounts that are effective for washing them, and the lather generated by massaging or rubbing with the hands may then be removed, after an optional leave-on time, by rinsing with water, the operation possibly being repeated one or more times.

### Process and use according to the invention

Another subject of the present invention relates to a process for washing and conditioning keratin fibres, in particular human keratin fibres such as the hair, comprising the application to said fibres of a composition according to the invention as defined above.

The composition may be applied to wet or dry hair, and preferably to wet or moist hair.

The composition is preferably applied to sensitized hair, especially sensitized hair.

According to one embodiment, the process consists in applying to keratin fibres an effective amount of the composition according to the invention, optionally massaging the fibres, optionally leaving the composition to stand on the fibres, and rinsing.

The leave-on time of the composition on the keratin fibres may be between a few seconds and 15 minutes and preferably between 30 seconds and 5 minutes. The composition is generally rinsed out with water.

An optional step of drying the keratin fibres may be performed.

The present invention also relates to the use of the composition according to the invention as described previously for washing and conditioning keratin fibres, in particular human keratin fibres such as the hair.

The examples that follow serve to illustrate the invention without, however, being limiting in nature.

### EXAMPLES

The compositions according to the invention are prepared from the ingredients indicated in the table below, the amounts of which are expressed as weight percentages relative to the total weight of the composition.

| | A (Reference) | B | C | D | E | F | G | Comp |
|---|---|---|---|---|---|---|---|---|
| Alkylpolyglucoside | - | - | - | - | - | - | - | 4 |
| Laureth-12 | - | 5 | 5 | 4 | 4 | 4 | 4 | - |
| Laureth-4 | 5 | 5 | 5 | 1 | 1 | 1 | 1 | 1 |
| Polysorbate 20 | 5 | - | - | - | - | - | - | - |
| Carboxylic acid Laureth-5 | 6.3 | 6.3 | 6.3 | 6 | 6 | 6 | 6 | 6 |
| Cocamidopropyl betaine | 3.8 | 3.8 | 3.8 | 7 | 7 | 7 | 7 | 7 |
| Sodium laureth sulfate | 1.75 | 1.75 | 1.75 | 7 | 7 | - | 7 | 7 |
| Sodium (C₁₄-C₁₆) olefin sulfonate | - | - | - | - | - | 7 | - | - |
| Polyquaternium-6 | 1 | 1 | 0.5 | 0.5 | 1 | 0.5 | 0.6 | 0.5 |
| Polyquaternium-53 | 0.5 | 0.5 | 0.25 | 0.25 | 0.5 | 0.25 | 0.25 | 0.25 |
| Polyquaternium-7 | - | - | - | - | - | - | 0.25 | - |
| Sodium chloride | - | 1 | 1 | 0.7 | 0.7 | 0.7 | 0.7 | 0.7 |
| Salicylic acid | 0.2 | 0.2 | 0.2 | 0.2 | 0.2 | 0.2 | 0.2 | 0.2 |
| Sodium benzoate | 0.5 | 0.5 | 0.5 | 0.5 | 0.5 | 0.5 | 0.5 | 0.5 |
| Sodium hydroxide/citric acid | qs pH = 5±0.5 | qs pH = 5±0.5 | qs pH = 5±0.5 | qs pH = 5±0.5 | qs pH = 6±0.5 | qs pH = 6±0.5 | qs pH = 5±0.5 | qs pH = 5±0.5 |
| Water | qs 100 | qs 100 | qs 100 | qs 100 | qs 100 | qs 100 | qs 100 | qs 100 |

The compositions are applied to locks of moderately sensitized hair (SA20) at a rate of 0.37 g of composition per gram of hair. They are then worked with the fingers five times and then rinsed ten times under a tap at a flow rate of 4 1/minute with water at 38°C.

A panel of 5 experts compared the feel and the ease of the disentangling on wet hair, of locks treated with compositions A to G relative to those treated with the comparative composition.

All the experts noted a better cosmetic quality of the feel and also better ease of the disentangling for formulations A to G compared with the comparative composition.

## Claims

1. Composition comprising:
(a) one or more anionic surfactants,
(b) a mixture of a first nonionic surfactant chosen from saturated or unsaturated, linear or branched, oxyalkylenated C₈ to C₄₀ fatty alcohols and of a second nonionic surfactant, different from the first nonionic surfactant, chosen from saturated or unsaturated, linear or branched, oxyalkylenated C₈ to C₄₀ fatty alcohols; the mixture of nonionic surfactants being present in a total content of at least 3% by weight relative to the total weight of the composition,
(c) one or more amphoteric surfactants in a total content of at least 2% by weight relative to the total weight of the composition, and
(d) one or more cationic polymers with a cationic charge density of greater than or equal to 4 meq./g.

2. Composition according to Claim 1, **characterized in that** the anionic surfactant(s) are chosen from anionic surfactants comprising in their structure one or more sulfate and/or sulfonate and/or phosphate groups, carboxylic alkyl ether anionic surfactants and/or mixtures thereof.

3. Composition according to Claim 2, **characterized in that** the anionic surfactant(s) comprising in their structure one or more sulfate and/or sulfonate and/or phosphate groups are chosen from alkyl sulfates, alkylamido sulfates, alkyl ether sulfates, alkylamido ether sulfates, alkylaryl ether sulfates, alkyl ether sulfosuccinates, acyl isethionates and methyl acyl taurates, olefin sulfonates, and salts thereof; the alkyl or acyl group of all these various compounds preferably containing from 8 to 24 carbon atoms, and the aryl group preferably denoting a phenyl or benzyl group.

4. Composition according to Claim 3, **characterized in that** the anionic surfactants are in the form of salts, and in particular alkaline salts, especially sodium salts, ammonium salts, amine salts, including amino alcohol salts, and/or magnesium salts.

5. Composition according to any one of the preceding claims, **characterized in that** the C₈-C₄₀ fatty alcohols comprise from 1 to 100 mol of ethylene oxide, in particular from 2 to 50 and more particularly from 2 to 40 mol of ethylene oxide.

6. Composition according to any one of the preceding claims, **characterized in that** the C₈ to C₄₀ fatty alcohols comprise from 3 to 20 mol of ethylene oxide and at least one C₈-C₂₀ alkyl chain.

7. Composition according to any one of the preceding claims, **characterized in that** the amphoteric surfactant(s) are chosen from (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines.

8. Composition according to any one of the preceding claims, **characterized in that** the cationic polymer(s) having a cationic charge density of greater than or equal to 4 meq./g are chosen from:
(1) homopolymers or copolymers derived from acrylic or methacrylic esters or amides and comprising at least one of the units of formula (V), (VI), (VII) or (VIII) below 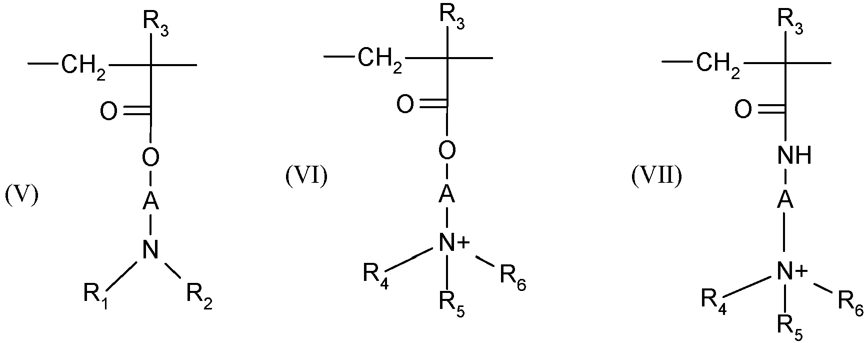 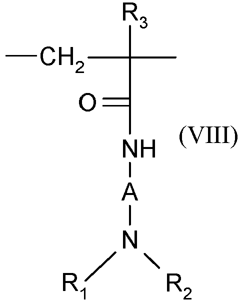 in which:
R₃, which may be identical or different, denote a hydrogen atom or a CH₃ radical;
A, which may be identical or different, represent a linear or branched alkyl group of 1 to 6 carbon atoms, preferably 2 or 3 carbon atoms, or a hydroxyalkyl group of 1 to 4 carbon atoms;
R₄, R₅ and R₆, which may be identical or different, represent an alkyl group containing from 1 to 18 carbon atoms or a benzyl radical and preferably an alkyl group containing from 1 to 6 carbon atoms;
R₁ and R₂, which may be identical or different, represent hydrogen or an alkyl group containing from 1 to 6 carbon atoms, and preferably methyl or ethyl;
X denotes an anion derived from a mineral or organic acid, such as a methosulfate anion or a halide such as chloride or bromide;
(2) Cyclopolymers of alkyldiallylamine or of dialkyldiallylammonium, such as homopolymers or copolymers comprising, as main constituent of the chain, units corresponding to formula (IX) or (X): 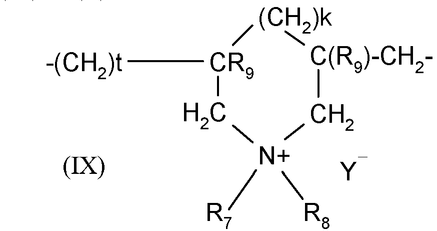 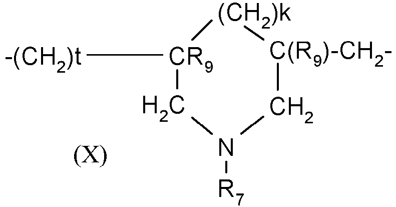
in which formulae k and t are equal to 0 or 1, the sum k + t being equal to 1;
R₉ denotes a hydrogen atom or a methyl radical;
R₇ and R₈, independently of each other, denote an alkyl group having from 1 to 6 carbon atoms, a hydroxyalkyl group in which the alkyl group contains preferably 1 to 5 carbon atoms, a lower (C₁-C₄) amidoalkyl group; or R₇ and R₈ may denote, together with the nitrogen atom to which they are attached, heterocyclic groups such as piperidyl or morpholinyl;
Y⁻ is an anion such as bromide, chloride, acetate, borate, citrate, tartrate, bisulfate, bisulfite, sulfate or phosphate;
(3) The quaternary diammonium polymer containing repeating units corresponding to formula (XI): 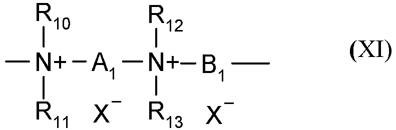 in which formula (XI):
R₁₀, R₁₁, R₁₂ and R₁₃, which may be identical or different, represent aliphatic, alicyclic or arylaliphatic radicals containing from 1 to 6 carbon atoms or lower hydroxyalkylaliphatic radicals, or alternatively R₁₀, R₁₁, R₁₂ and R₁₃, together or separately, constitute, with the nitrogen atoms to which they are attached, heterocycles optionally containing a second heteroatom other than nitrogen, or alternatively R₁₀, R₁₁, R₁₂ and R₁₃ represent a linear or branched C₁-C₆ alkyl radical substituted with a nitrile, ester, acyl or amide group or a group -CO-O-R₁₄-D or -CO-NH-R₁₄-D where R₁₄ is an alkylene and D is a quaternary ammonium group;
A1 and B1 represent polymethylene groups containing from 2 to 8 carbon atoms, which may be linear or branched and saturated or unsaturated and may contain, bonded to or intercalated in the main chain, one or more aromatic rings, or one or more oxygen or sulfur atoms, or sulfoxide, sulfone, disulfide, amino, alkylamino, hydroxyl, quaternary ammonium, ureido, amide or ester groups, and
X⁻ denotes an anion derived from a mineral or organic acid;
A1, R₁₀ and R₁₂ can form, with the two nitrogen atoms to which they are attached, a piperazine ring; in addition, if A1 denotes a linear or branched, saturated or unsaturated alkylene or hydroxyalkylene radical, B1 can also denote a group -(CH₂)n-CO-D-OC-(CH₂)n- in which D denotes:
a) a glycol residue of formula: -O-Z-O-, where Z denotes a linear or branched hydrocarbon-based radical or a group corresponding to one of the following formulae:
-(CH₂-CH₂-O)x-CH₂-CH₂-
-[CH₂-CH(CH₃)-O]y-CH₂-CH(CH₃)-
where x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula: -NH-Y-NH-, where Y denotes a linear or branched hydrocarbon-based radical, or else the divalent radical -CH₂-CH₂-S-S-CH₂-CH₂-;
d) a ureylene group of formula: -NH-CO-NH-;
(4) Polyquaternary ammonium polymers formed from repeating units of formula (XII): 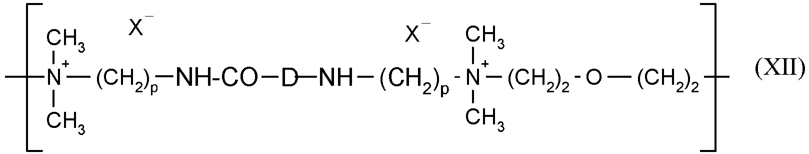 in which p denotes an integer ranging from 1 to 6 approximately, D may be nothing or may represent a group -(CH₂)ᵣCO- in which r denotes a number equal to 4 or 7, and X⁻ is an anion;
(5) Quaternary polymers of vinylpyrrolidone and of vinylimidazole.

9. Composition according to any one of the preceding claims, **characterized in that** the cationic polymers having a charge density of greater than or equal to 4 meq./g are chosen from dialkyldiallylammonium halide homopolymers.

10. Process for washing and conditioning keratin fibres, in particular human keratin fibres such as the hair, comprising the application to said fibres of a composition according to the invention as defined in any one of Claims 1 to 9.

11. Use of the composition according to the invention as defined according to any one of Claims 1 to 9, for washing and conditioning keratin fibres, in particular human keratin fibres such as the hair.

## Patentansprüche

1. Zusammensetzung, umfassend:
(a) ein oder mehrere anionische Tenside,
(b) eine Mischung von einem ersten nichtionischen Tensid, das aus gesättigten oder ungesättigten, linearen oder verzweigten, oxyalkylenierten C₈- bis C₄₀-Fettalkoholen ausgewählt ist, und einem zweiten nichtionischen Tensid, das von dem ersten nichtionischen Tensid verschieden ist und aus gesättigten oder ungesättigten, linearen oder verzweigten, oxyalkylenierten C₈- bis C₄₀-Fettalkoholen; wobei die Mischung von nichtionischen Tensiden in einem Gesamtgehalt von mindestens 3 Gew.-%, bezogen auf das Gesamtgewicht der Zusammensetzung, vorliegt,
(c) ein oder mehrere amphotere Tenside in einem Gesamtgehalt von mindestens 2 Gew.-%, bezogen auf das Gesamtgewicht der Zusammensetzung, und
(d) ein oder mehrere kationische Polymere mit einer Kationenladungsdichte größer oder gleich 4 meq/g.

2. Zusammensetzung nach Anspruch 1, **dadurch gekennzeichnet, dass** das anionische Tensid bzw. die anionischen Tenside aus anionischen Tensiden, die in ihrer Struktur eine oder mehrere Sulfat- und/oder Sulfonat- und/oder Phosphatgruppen enthalten, anionischen Carboxylalkylether-Tensiden und/oder Mischungen davon ausgewählt ist bzw. sind.

3. Zusammensetzung nach Anspruch 2, **dadurch gekennzeichnet, dass** das anionische Tensid bzw. die anionischen Tenside, das bzw. die in ihrer Struktur eine oder mehrere Sulfat- und/oder Sulfonat- und/oder Phosphatgruppen enthält bzw. enthalten, aus Alkylsulfaten, Alkylamidosulfaten, Alkylethersulfaten, Alkylamidoethersulfaten, Alkylarylethersulfaten, Alkylethersulfosuccinaten, Acylisethionaten und Methylacyltauraten, Olefinsulfonaten und Salzen davon ausgewählt ist bzw. sind, wobei die Alkyl- bzw. Acylgruppe aller dieser verschiedenen Verbindungen vorzugsweise 8 bis 24 Kohlenstoffatome enthält und die Arylgruppe vorzugsweise eine Phenyl- oder Benzylgruppe bedeutet.

4. Zusammensetzung nach Anspruch 3, **dadurch gekennzeichnet, dass** die anionischen Tenside in Form von Salzen und insbesondere Alkalisalzen, insbesondere Natriumsalzen, Ammoniumsalzen, Aminsalzen einschließlich Aminoalkoholsalzen und/oder Magnesiumsalzen, vorliegen.

5. Zusammensetzung nach einem der vorhergehenden Ansprüche, **dadurch gekennzeichnet, dass** die C₈- bis C₄₀-Fettalkohole 1 bis 100 Mol Ethylenoxid, insbesondere 2 bis 50 und spezieller 2 bis 40 Mol Ethylenoxid enthalten.

6. Zusammensetzung nach einem der vorhergehenden Ansprüche, **dadurch gekennzeichnet, dass** die C₈- bis C₄₀-Fettalkohole 3 bis 20 Mol Ethylenoxid und mindestens eine C₈-C₂₀-Alkylkette enthalten.

7. Zusammensetzung nach einem der vorhergehenden Ansprüche, **dadurch gekennzeichnet, dass** das amphotere Tensid bzw. die amphoteren Tenside aus (C₈-C₂₀) -Alkylbetainen, (C₈-C₂₀) -Alkylsulfobetainen, (C₈-C₂₀) -Alkylamido- (C₃-C₈) -alkylbetainen und (C₈-C₂₀) -Alkylamido- (C₆-C₈) -alkylsulfobetainen ausgewählt ist bzw. sind.

8. Zusammensetzung nach einem der vorhergehenden Ansprüche, **dadurch gekennzeichnet, dass** das kationische Polymer bzw. die kationischen Polymere mit einer Kationenladungsdichte größer oder gleich 4 meq/g aus
(1) Homopolymeren oder Copolymeren, die sich von Acrylsäure- oder Methacrylsäureestern oder -amiden ableiten und mindestens eine der Einheiten der nachstehenden Formeln (V), (VI), (VII) oder (VIII) umfassen: 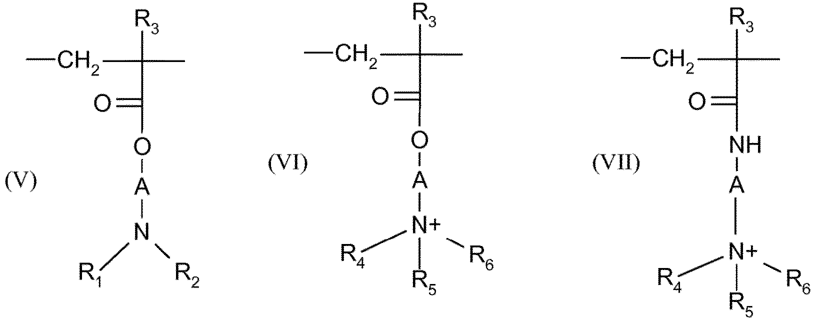 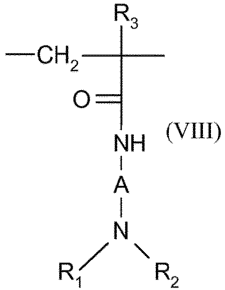 in denen:
R₃, das gleich oder verschieden sein kann, ein Wasserstoffatom oder einen CH₃-Rest bedeutet;
A, das gleich oder verschieden sein kann, für eine lineare oder verzweigte Alkylgruppe mit 1 bis 6 Kohlenstoffatomen, vorzugsweise 2 oder 3 Kohlenstoffatomen, oder eine Hydroxyalkylgruppe mit 1 bis 4 Kohlenstoffatomen steht;
R₄, R₅ und R₆, die gleich oder verschieden sein können, für eine Alkylgruppe mit 1 bis 18 Kohlenstoffatomen oder einen Benzylrest und
vorzugsweise eine Alkylgruppe mit 1 bis 6 Kohlenstoffatomen stehen;
R₁ und R₂, die gleich oder verschieden sein können, für Wasserstoff oder eine Alkylgruppe mit 1 bis 6 Kohlenstoffatomen und vorzugsweise Methyl oder Ethyl stehen;
X ein Anion bedeutet, das sich von einer Mineralsäure oder organischen Säure ableitet, wie ein Methosulfat-Anion oder ein Halogenid wie Chlorid oder Bromid,
(2) Cyclopolymeren von Alkyldiallylamin oder Dialkyldiallylammonium, wie Homopolymeren oder Copolymeren, die als Hauptbestandteil der Kette Einheiten umfassen, die der Formel (IX) oder (X) entsprechen: 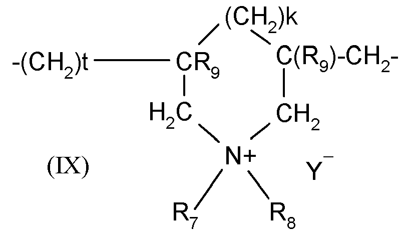 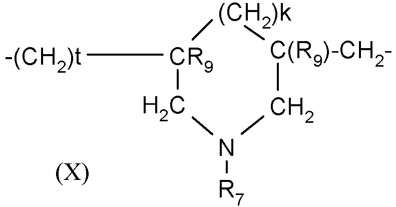
in denen k und t gleich 0 oder 1 sind, die Summe k + t gleich 1 ist;
R₉ ein Wasserstoffatom oder einen Methylrest bedeutet;
R₇ und R₈ unabhängig voneinander eine Alkylgruppe mit 1 bis 6 Kohlenstoffatomen, eine Hydroxyalkylgruppe, in der die Alkylgruppe vorzugsweise 1 bis 5 Kohlenstoffatome enthält, eine niedere (C₁-C₄)-Amidoalkylgruppe bedeuten oder R₇ und R₈ zusammen mit dem Stickstoffatom, an das sie gebunden sind, heterocyclische Gruppen wie Piperidyl oder Morpholinyl bedeuten können;
Y⁻ für ein Anion wie Bromid, Chlorid, Acetat, Borat, Citrat, Tartrat, Hydrogensulfat, Hydrogensulfit, Sulfat oder Phosphat steht;
(3) den quaternären Diammoniumpolymeren mit Wiederholungseinheiten, die der folgenden Formel (XI) entsprechen: 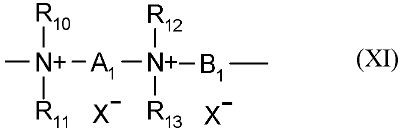 wobei in der Formel (XI):
R₁₀, R₁₁, R₁₂ und R₁₃, die gleich oder verschieden sein können, für aliphatische, alicyclische oder arylaliphatische Reste mit 1 bis 6 Kohlenstoffatomen oder niedere hydroxyalkylaliphatische Reste stehen oder alternativ dazu R₁₀, R₁₁, R₁₂ und R₁₃ zusammen oder separat mit den Stickstoffatomen, an die sie gebunden sind, Heterocyclen bilden, die gegebenenfalls außer Stickstoff ein zweites Heteroatom enthalten, oder alternativ dazu R₁₀, R₁₁, R₁₂ und R₁₃ für einen linearen oder verzweigten C₁-C₆-Alkylrest, der durch eine Nitril-, Ester-, Acyl- oder Amidgruppe oder eine -CO-O-R₁₄-D- oder -CO-NH-R₁₄-D-Gruppe substituiert ist, stehen, wobei R₁₄ für ein Alkylen steht und D für eine quaternäre Ammoniumgruppe steht;
A1 und B1 für Polymethylengruppen mit 2 bis 8 Kohlenstoffatomen stehen, die linear oder verzweigt und gesättigt oder ungesättigt sein können und an der Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können;
X⁻ ein Anion bedeutet, das sich von einer Mineralsäure oder organischen Säure ableitet;
A1, R₁₀ und R₁₂ mit den beiden Stickstoffatomen, an die sie gebunden sind, einen Piperazinring bilden können; außerdem B1 dann, wenn A1 einen linearen oder verzweigten, gesättigten oder ungesättigten Alkylen- oder Hydroxyalkylenrest bedeutet, auch eine -(CH₂)ₙ-CO-D-OC(CH₂)ₙ-Gruppe bedeuten kann, in der D:
a) einen Glykolrest der Formel: -O-Z-O-, wobei Z für einen linearen oder verzweigten Rest auf Kohlenwasserstoffbasis oder eine Gruppe, die einer der folgenden Formeln entspricht, steht:
-(CH₂-CH₂-O)x-CH₂-CH₂-
-[CH₂-CH(CH₃)-O]y-CH₂-CH(CH₃)-,
wobei x und y eine ganze Zahl von 1 bis 4 bedeuten und einen definierten und einzigartigen Polymerisationsgrad wiedergeben oder für eine beliebige Zahl von 1 bis 4 stehen und einen durchschnittlichen Polymerisationsgrad wiedergeben;
b) einen bissekundären Diaminrest, wie ein Piperazinderivat;
c) einen bisprimären Diaminrest der Formel: -NH-Y-NH-, wobei Y einen linearen oder verzweigten Kohlenwasserstoffrest oder auch den zweiwertigen Rest -CH₂-CH₂-S-S-CH₂-CH₂- bedeutet;
d) eine Ureylengruppe der Formel: -NH-CO-NHbedeutet;
(4) polyquaternären Ammoniumpolymeren, die aus Wiederholungse inheiten der Formel (XII) bestehen: 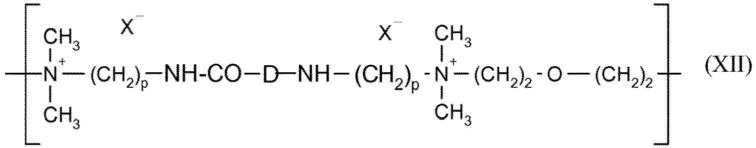 in der p eine ganze Zahl im Bereich von ungefähr 1 bis 6 bedeutet, D nichts sein kann oder für eine Gruppe -(CH₂)ᵣ-CO- stehen kann, wobei r für eine Zahl mit einem Wert von 4 oder 7 steht, und X⁻ für ein Anion steht;
(5) quaternären Polymeren von Vinylpyrrolidon und Vinylimidazol
ausgewählt ist bzw. sind.

9. Zusammensetzung nach einem der vorhergehenden Ansprüche, **dadurch gekennzeichnet, dass** die kationischen Polymere mit einer Ladungsdichte größer oder gleich 4 meq/g aus Dialkyldiallylammoniumhalogenid-Homopolymeren ausgewählt sind.

10. Verfahren zum Waschen und Konditionieren von Keratinfasern, insbesondere menschlichen Keratinfasern wie dem Haar, bei dem man auf die Fasern eine erfindungsgemäße Zusammensetzung gemäß einem der Ansprüche 1 bis 9 aufbringt.

11. Verwendung der erfindungsgemäßen Zusammensetzung gemäß einem der Ansprüche 1 bis 9 zum Waschen und Konditionieren von Keratinfasern, insbesondere menschlichen Keratinfasern wie dem Haar.

## Revendications

1. Composition comprenant :
(a) un ou plusieurs tensioactifs anioniques,
(b) un mélange d'un premier tensioactif non ionique choisi parmi les alcools gras en C₈ à C₄₀, saturés ou non, linéaires ou ramifiés, oxyalkylénés, et d'un deuxième tensioactif non ionique, différent du premier tensioactif non ionique, choisi parmi les alcools gras en C₈ à C₄₀, saturés ou non, linéaires ou ramifiés, oxyalkylénés ; le mélange de tensioactifs non ioniques étant présent dans une teneur totale d'au moins 3% en poids par rapport au poids total de la composition,
(c) un ou plusieurs tensioactifs amphotères dans une teneur totale d'au moins 2% en poids par rapport au poids total de la composition, et
(d) un ou plusieurs polymères cationiques ayant une densité de charge cationique supérieure ou égale à 4 meq/g.

2. Composition selon la revendication 1, **caractérisée en ce que** le ou les tensioactifs anioniques est ou sont choisis parmi les tensioactifs anioniques comportant dans leur structure un ou plusieurs groupes sulfates et/ou sulfonates et/ou phosphates, les tensioactifs anioniques alkyl éther carboxyliques et/ou leurs mélanges.

3. Composition selon la revendication 2, **caractérisée en ce que** les tensioactifs anioniques comportant dans leur structure un ou plusieurs groupes sulfates et/ou sulfonates et/ou phosphates sont choisis parmi les alkylsulfates, les alkylamidosulfates, les alkyl éther sulfates, les alkylamidoéthersulfates, les alkylaryléthersulfates, les alkyléthersulfosuccinates, les acyl iséthionates, les méthyl acyl taurates, les oléfines sulfonates et leurs sels ; le groupe alkyle ou acyle de tous ces différents composés comportant de préférence de 8 à 24 atomes de carbone, et le groupe aryle désignant de préférence un groupement phényle ou benzyle.

4. Composition selon la revendication 3, **caractérisée en ce que** les tensioactifs anioniques se trouvent sous la forme de sels, et en particulier de sels alcalins, notamment de sels de sodium, de sels d'ammonium, de sels d'amines dont les sels d'aminoalcools, et/ou de sels de magnésium.

5. Composition selon l'une quelconque des revendications précédentes, **caractérisée en ce que** les alcools gras en C₈-C₄₀ comprennent de 1 à 100 moles d'oxyde d'éthylène, en particulier de 2 à 50, et plus particulièrement de 2 à 40 moles d'oxyde d'éthylène.

6. Composition selon l'une quelconque des revendications précédentes, **caractérisée en ce que** les alcools gras en C₈ à C₄₀ comprennent de 3 à 20 moles d'oxyde d'éthylène et au moins une chaîne alkyle en C₈-C₂₀.

7. Composition selon l'une quelconque des revendications précédentes, **caractérisée en ce que** le ou les tensioactifs amphotères est ou sont choisis parmi les alkyl(C₈-C₂₀)bétaïnes, les alkyl(C₈-C₂₀)sulfobétaïnes, les alkyl(C₈-C₂₀)amidoalkyl(C₃-C₈)bétaïnes et les alkyl(C₈-C₂₀)amidalkyl(C₆-C₈)sulfobétaïnes.

8. Composition selon l'une quelconque des revendications précédentes, **caractérisée en ce que** le ou les polymères cationiques ayant une densité de charge cationique supérieure ou égale à 4 meq/g sont choisis parmi :
(1) les homopolymères ou copolymères dérivés d'esters ou d'amides acryliques ou méthacryliques et comportant au moins un des motifs de formules (V), (VI), (VII) ou (VIII) suivantes 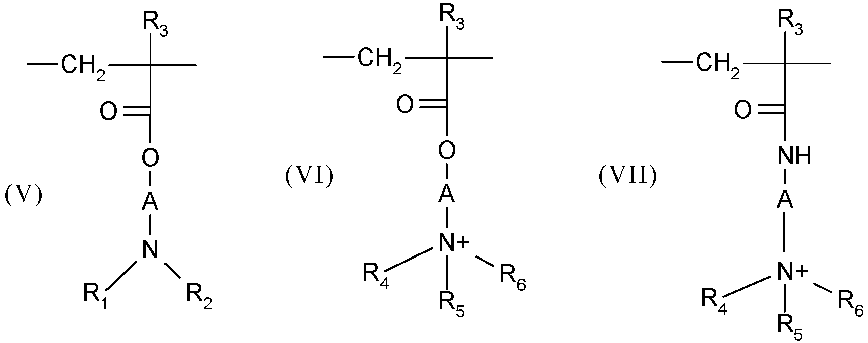 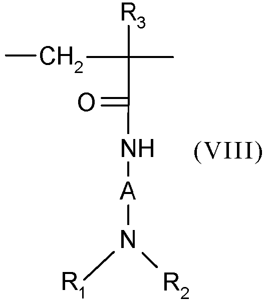 dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
R₁ et R₂, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure ;
(2) Les cyclopolymères d'alkyl diallyl amine ou de dialkyl diallyl ammonium tels que les homopolymères ou copolymères comportant comme constituant principal de la chaîne des motifs répondant aux formules (IX) ou (X): 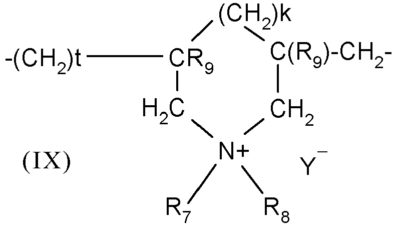 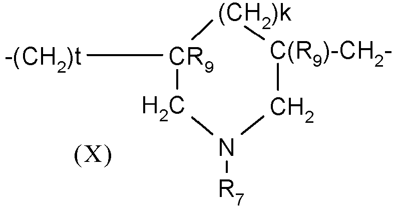
formules dans lesquelles k et t sont égaux à 0 ou 1, la somme k + t étant égale à 1 ;
R₉ désigne un atome d'hydrogène ou un radical méthyle ;
R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ;
Y⁻ est un anion tel que bromure, chlorure, acétate, borate, citrate, tartrate, bisulfate, bisulfite, sulfate, phosphate ;
(3) Le polymère de diammonium quaternaire contenant des motifs récurrents répondant à la formule (XI) : 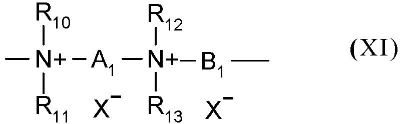 formule (XI) dans laquelle :
R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃, représentent un radical alkyle en C₁-C₆ linéaire ou ramifié substitué par un groupement nitrile, ester, acyle, amide ou -CO-O-R₁₄-D ou -CO-NH-R₁₄-D où R₁₄ est un alkylène et D un groupement ammonium quaternaire ;
A1 et B1 représentent des groupements polyméthyléniques contenant de 2 à 8 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)ₙ-CO-D-OC-(CH₂)ₙ- dans lequel D désigne :
a) un reste de glycol de formule : -O-Z-O-, où Z désigne un radical hydrocarboné linéaire ou ramifié ou un groupement répondant à l'une des formules suivantes :
-(CH₂-CH₂-O)ₓ-CH₂-CH₂-
-[CH₂-CH(CH₃)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
d) un groupement uréylène de formule : -NH-CO-NH- ;
(4) Les polyammoniums quaternaires constitués de motifs récurrents de formule (XII): 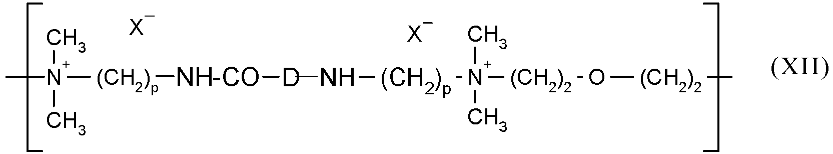 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
(5) Les polymères quaternaires de vinylpyrrolidone et de vinylimidazole.

9. Composition selon l'une quelconque des revendications précédentes, **caractérisée en ce que** les polymères cationiques ayant une densité de charge supérieure ou égale à 4 meq/g sont choisis parmi les homopolymères d'halogénures dialkyldiallylammonium.

10. Procédé pour le lavage et le conditionnement des fibres kératiniques, en particulier des fibres kératiniques humaines telles que les cheveux, comprenant l'application sur lesdites fibres d'une composition selon l'invention telle que définie selon l'une quelconque des revendications 1 à 9.

11. Utilisation de la composition selon l'invention telle que définie selon l'une quelconque des revendications 1 à 9 pour le lavage et le conditionnement des fibres kératiniques, en particulier des fibres kératiniques humaines telles que les cheveux.
